(19) Europäisches Patentamt
European Patent Office
Office européen des brevets

(11) EP 1 468 704 B1

(12) **EUROPEAN PATENT SPECIFICATION**

(45) Date of publication and mention of the grant of the patent:
**28.02.2007 Bulletin 2007/09**

(51) Int Cl.:
***A61L 29/04*** *(2006.01)*

(21) Application number: **04008216.6**

(22) Date of filing: **05.04.2004**

# (54) Medical member mainly comprising syndiotactic 1,2-polybutadiene

Medizinischer Artikel auf Basis von syndiotaktischem 1,2-Polybutadien

Article médical à base de 1,2-polybutadiène syndiotactique

(84) Designated Contracting States:
**DE**

(30) Priority: **11.04.2003 JP 2003107406**

(43) Date of publication of application:
**20.10.2004 Bulletin 2004/43**

(73) Proprietor: **JSR Corporation**
**Tokyo 104-8410 (JP)**

(72) Inventors:
- **Furuichi, Minoru, c/o JSR Corp.**
  **Tokyo 104-8410 (JP)**
- **Okada, Kouji, c/o JSR Corp.**
  **Tokyo 104-8410 (JP)**
- **Aoyama, Teruo, c/o JSR Corp.**
  **Tokyo 104-8410 (JP)**

(74) Representative: **TBK-Patent**
**Bavariaring 4-6**
**80336 München (DE)**

(56) References cited:
**EP-A- 1 123 715**
**EP-A- 1 243 285**
**WO-A-03/018685**

- **PATENT ABSTRACTS OF JAPAN vol. 0150, no. 92 (C-0811), 6 March 1991 (1991-03-06) & JP 2 305834 A (TERUMO CORP), 19 December 1990 (1990-12-19)**

EP 1 468 704 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

BACKGROUND OF THE INVENTION

(1) FIELD OF THE INVENTION

The present invention relates to a medical member comprising a tube mainly comprising syndiotactic 1,2-polybutadiene and a connecting member such as a connector connected thereto which mainly comprises the above-mentioned polybutadiene, and a medical instrument using the same. More particularly, the present invention relates to a medical member mainly comprising syndiotactic 1,2-polybutadiene, which is excellent in flexibility and hardness, and also has steam sterilization resistance, and a medical instrument using the same.

(2) DESCRIPTION OF THE RELATED ART

Syndiotactic 1,2-polybutadiene is a thermoplastic elastomer having both the property of a plastic (hardness) and the properties of rubber (elasticity and flexibility), and can be easily formed by use of a general-purpose polymer processing machine, while having a definite crystallinity. Accordingly, it has come to be used in various industrial goods. In particular, it is excellent in gas permeability resistance and transparency. Compared to vinyl chloride resins requiring a large amount of plasticizers which cause an environmental problem such as a problem of environmental hormones, it can be shaped without the addition of plasticizers, and has moderate flexibility and self-adhesion. Accordingly, the application thereof to medical instruments such as infusion tubes and catheters is expanding.

Further, syndiotactic 1,2-polybutadiene has a melt-starting temperature as low as 70 to 90˚C, when low in crystallinity. When this is used in medical instruments such as infusion tubes, fusion solution containers and catheters, and sterilized by steam, a problem is practically encountered because of its poor heat resistance. Accordingly, sterilization by ethylene oxide gas has been employed.

As a method for overcoming the lack of the balance in performance, while keeping the characteristic of syndiotactic 1,2-polybutadiene that it is a thermoplastic elastomer having both the property of a plastic (hardness) and the properties of rubber (elasticity and flexibility), there has been proposed a method of crosslinking only a surface of a formed article by irradiating it with an ultraviolet ray having a specific wavelength, thereby curing it (Japanese Patent Laid-Open Publication 2000-129017). This method exhibits a reasonable effect in respect to improvement of the balance in performance among flexibility, transparency and heat resistance (high-pressure steam sterilization resistance).

Further, in order to obtain a harder surface, various electron beam irradiation methods have also been proposed. These methods exhibit a definite effect for scratch resistance.

However, in the formed article obtained by the method described in the above-mentioned patent specification, only the surface layer is crosslinked by the irradiation of the ultraviolet ray having a wavelength within the specific region. It is therefore difficult to homogeneously crosslink, for example, a formed article having an irregular shape such as a tube. Further, the heat resistance of the inside of a medical tube is not necessarily fully satisfactory. When the tube is treated by ordinary high-pressure steam sterilization (121˚C X 20 minutes), there is the possibility that the inside insufficiently crosslinked melts to cause the nonuniformity of the tube thickness, that is to say, variation in hardness of the tube. On the other hand, according to the conventional electron beam irradiation methods, the inside of the formed article is also hardened in excess, which poses the problem that flexibility, one property of syndiotactic 1,2-polybutadiene, is almost lost.

Further, a medical instrument such as an infusion set is constituted, for example, by joining the above-mentioned medical tube to a connecting member. The use of syndiotactic 1,2-polybutadiene in the medical tube from the viewpoint of no inclusion of plasticizers as described above results in insufficient heat resistance or adhesion with a different type of material, because an olefinic resin such as polypropylene or a polycarbonate resin is generally employed as the connecting member. As a result, the joining or adhesion strength between the tube and the connecting member becomes insufficient, which is liable to cause a leak.

SUMMARY OF THE INVENTION

It is therefore an object of the present invention to provide a medical member mainly comprising syndiotactic 1,2-polybutadiene, which is useful for medical use, is excellent in flexibility and hardness, has no leak at a joint, and is also excellent in steam sterilization resistance.

Another object of the invention is to provide a medical instrument using the above-mentioned medical member.

According to the present invention, there is provided a medical member comprising a tube mainly comprising syndiotactic 1,2-polybutadiene having a crystallinity of 5 to 25 % and a connecting member connected thereto, the connecting member mainly comprising syndiotactic 1,2-polybutadiene having a crystallinity of 25 to 40% and being at

least one selected from the group consisting of a closure-piercing device, a drip chamber, a graduated buret, an air trap, an injection site, a three-way cock and a connector.

The term "mainly comprising syndiotactic 1,2-polybutadiene used herein means not only syndiotactic 1,2-polybutadiene alone but also a composition containing (A) 100 to 60 parts by weight of syndiotactic 1,2-polybutadiene and (B) 0 to 40 parts by weight of at least one different thermoplastic polymer selected from the group consisting of polyethylene, polypropylene, a styrene-butadiene-styrene block copolymer (SBS), a styrene-isoprene-styrene block copolymer (SIS), hydrogenated polymers thereof (SEBS and SEPS), polybutadiene (BR) other than the above-mentioned syndiotactic 1,2-polybutadiene, an ABS resin, polyisoprene, various polyethylenes (LLDPE, ULDPE and LDPE), an ethylene-vinyl acetate copolymer, an ethylene-acrylate copolymer and an ethylene-methacrylate copolymer.

Further, in the medical member of the present invention, the tube is connected to the connecting member by solvent adhesion, ultrasonic adhesion or high-frequency adhesion.

Still further, the medical member of the present invention includes one sterilizable by steam.

Furthermore, in the medical member of the present invention, it is preferred that the tube is crosslinked by electron beam irradiation.

Besides, it is preferred that the medical member of the present invention has a halogen content of 200 ppm or less.

In addition, the medical member of the present invention may contain a lubricant in an amount of 10 parts by weight or less based on 100 parts by weight of a resin component mainly comprising syndiotactic 1,2-polybutadiene.

Then, according to the present invention, there is provided a medical instrument having the above-mentioned medical member as a constituent element.

## BRIEF DESCRIPTION OF THE DRAWINGS

Fig. 1 is a plan view showing an infusion set having medical members of the present invention as constituent elements; and

Fig. 2 is a schematic view showing (a) a connector and (b) a tube.

## DESCRIPTION OF THE PREFERRED EMBODIMENTS

In the tube and connecting member employed in the present invention, there is used (A) syndiotactic 1,2-polybutadiene alone or a composition of (A) syndiotactic 1,2-polybutadiene and (B) an additional thermoplastic polymer. The crystallinity and melting point within these ranges result in an excellent balance between mechanical strength such as tensile strength or tear strength and flexibility.

Syndiotactic 1, 2-polybutadiene having a crystallinity of about 5 to about 25% (hereinafter also referred to as "low crystalline RB") is excellent in flexibility, so that it is used as a main body of the tube. For this application, the low crystalline RB having a crystallinity of from 15 to 25 % (melting point: about 70 to 95˚C) is more preferable. However, the low crystalline RB is inferior in steam sterilization resistance because of its low melting point. It is therefore desirable to crosslink it by electron beam irradiation, thereby giving heat resistance, as described later.

On the other hand, syndiotactic 1,2-polybutadiene having a crystallinity of about 25 to about 40% (hereinafter also referred to as "high crystalline RB") is relatively high in melting point (about 96 to about 150˚C), while it is high in hardness and therefore inferior in flexibility. Accordingly, it is preferably used as the connecting member rather than as the tube in the present invention. For the application to the connecting member, the high crystalline RB having the crystallinity of about 28% to 38%(melting point: about 100 to 140˚C)is more preferable.

For example, syndiotactic 1,2-polybutadiene having a 1,2-bond content of 70% or more is used as (A) the syndiotactic 1,2-polybutadiene in the present invention, and obtained, for example, by polymerizing butadiene in the presence of a catalyst containing a cobalt compound and an aluminoxane. However, the production method should not be construed as being limited thereto.

The 1,2-bond content in butadiene bond units of (A) the syndiotactic 1,2-polybutadiene used in the present invention is usually 70% or more, preferably 80% or more, and more preferably 90% or more. When the 1,2-bond content is 70% or more, the 1,2-polybutadiene exhibits properties as a good thermoplastic elastomer.

(A) The syndiotactic 1,2-polybutadiene used in the present invention may be copolymerized with a small amount of a conjugated diene other than butadiene. The conj ugated dienes other than butadiene include 1,3-pentadiene, a higher alkyl group-substituted 1,3-butadiene derivative and a 2-alkyl-substituted 1,3-butadiene.

Of these, the higher alkyl group-substituted 1,3-butadiene derivatives include 1-pentyl-1,3-butadiene, 1-hexyl-1,3-butadiene, 1-heptyl-1,3-butadiene and 1-octyl-1,3-butadiene.

Here, typical examples of the 2-alkyl-substituted 1,3-butadienes include 2-methyl-1,3-butadiene (isoprene), 2-ethyl-1,3-butadiene, 2-propyl-1,3-butadiene, 2-isopropyl-1,3-butadiene, 2-butyl-1,3-butadiene, 2-isobutyl-1,3-butadiene, 2-amyl-1,3-butadiene, 2-isoamyl-1,3-butadiene, 2-hexyl-1,3-butadiene, 2-cyclohexyl-1,3-butadiene, 2-isohexyl-1,3-butadiene, 2-heptyl-1,3-butadiene, 2-isoheptyl-1,3-butadiene, 2-octyl-1, 3-butadiene and 2-isooctyl-1,3-butadiene. Of these conjugated dienes, preferred examples of the conjugated dienes to be copolymerized with butadiene include isoprene and 1,3-pentadiene. The content of butadiene in monomer components subjected to polymerization is preferably 50 mol% or more, and particularly 70 mol% or more.

As described above, (A) the syndiotactic 1,2-polybutadiene used in the present invention is obtained, for example, by polymerizing butadiene in the presence of the catalyst containing the cobalt compound and the aluminoxane. The above-mentioned cobalt compounds include preferably an organic acid salt of cobalt having 4 or more carbon atoms. Specific examples of the organic acid salts of cobalt include a butyrate; a hexanoate; a heptylate; an octylate such as 2-ethyl-hexylate; a decanoate; a salt of a higher fatty acid such as stearate, oleate or erucate; a benzoate; an alkyl-, aralkyl- or allyl-substituted benzoate such as a tolylate, a xylylate or an ethylbenzoate; a naphthoate; and an alkyl-, aralkyl- or allyl-substituted naphthoate. Of these, 2-ethylhexylate or a so-called octylate, a stearate and a benzoate are preferred for excellent solubility in a hydrocarbon solvent.

The above-mentioned aluminoxanes include, for example, one represented by the following general formula (I) or general formula (II):

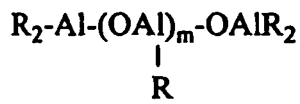

-------(I)

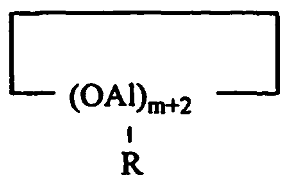

-----(II)

In the aluminoxane represented by general formula (I) or (II), R is a hydrocarbon group such as a methyl group, an ethyl group, a propyl group or a butyl group, preferably a methyl group or an ethyl group, and particularly preferably a methyl group. Further, m is an integer of 2 or more, preferably an integer of 5 or more, and more preferably an integer of 10 to 100. Specific examples of the aluminoxanes include methylaluminoxane, ethylaluminoxane, propylaluminoxane and butyl-aluminoxane, and methylaluminoxane is particularly preferred.

It is very preferred that the polymerization catalyst contains a phosphine compound, in addition to the above-mentioned cobalt compound and aminoxane. The phosphine compound is a component effective for activation of the polymerization catalyst, and the control of the vinyl bond structure and crystallinity. It preferably includes an organic phosphorus compound represented by the following general formula (III):

$$P(Ar)_n(R')_{3-n} \qquad (III)$$

In general formula (III), Ar represents a group shown below:

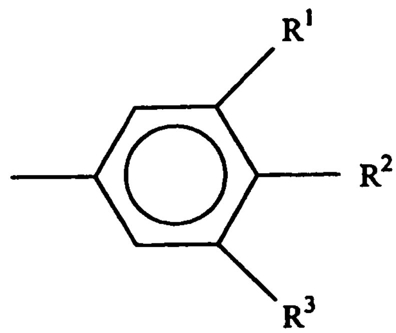

wherein, $R^1$, $R^2$ and $R^3$, which may be the same or different, each represent a hydrogen atom, an alkyl group preferably having 1 to 6 carbon atoms, a halogen atom, an alkoxyl group preferably having 1 to 6 carbon atoms or an aryl group preferably having 6 to 12 carbon atoms.

Further, in general formula (III), R' represents a cycloalkyl group or an alkyl-substituted cycloalkyl group, and n is an integer of 0 to 3.

Specific examples of the phosphine compounds represented by general formula (III) include tri (3-methylphenyl) phosphine, tri(3-ethylphenyl)phosphine, tri(3,5-dimethylphenyl)phosphine, tri(3,4-dimethylphenyl)phosphine, tri(3-isopropylphenyl)phosphine, tri(3-t-butylphenyl)phosphine, tri(3,5-diethylphenyl)phosphine, tri(3-methyl-5-ethylphenyl) phosphine, tri (3-phenylphenyl)phosphine, tri (3,4,5-trimethylphenyl)phosphine, tri(4-methoxy-3,5-dimethylphenyl)phosphine, tri(4-ethoxy-3,5-diethylphenyl)phosphine, tri(4-butoxy-3,5-dibutylphenyl) phosphine, tri(p-methoxyphenyl)-phosphine, tricyclohexylphosphine, dicyclohexylphenylphosphine, tribenzylphosphine, tri(4-methylphenylphosphine) and tri (4-ethylphenylphosphine). Of these, particularly preferred examples thereof include triphenylphosphine, tri-(3-methylphenyl)phosphine and tri(4-methoxy-3,5-dimethylphenyl)phosphine.

Further, as the cobalt compound, there can be used a compound represented by the following general formula (IV):

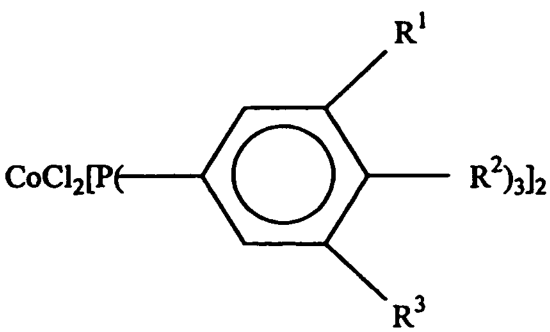 ------(IV)

The compound represented by the above-mentioned general formula (IV) is a complex having a phosphine compound in which n is 3 in the above-mentioned general formula (III), as a ligand to cobalt chloride. When this cobalt compound is used, one previously synthesized may be used, or a method of contacting cobalt chloride with the phosphine compound in a polymerization system may be used. The amount of 1,2-vinyl bonds and crystallinity of the resulting syndiotactic 1,2-polybutadiene can be controlled by variously selecting the phosphine compound in the complex.

Specific examples of the cobalt compounds represented by the above-mentioned general formula (IV) include cobalt bis(triphenylphosphine)dichloride, cobalt bis[tris(3-methyl-phenylphosphine)]dichloride, cobalt bis[tris(3-ethylphenylphosphine)]dichloride, cobalt bis[tris(9-methyl-phenylphosphine)]dichloride, cobalt bis[tris(3,5-dimethyl-phenylphosphine)]dichloride, cobalt bis[tris(3,4-dimethyl-phenylphosphine)]dichloride, cobalt bis[tris(3-isopropyl-phenylphosphine)]dichloride, cobalt bis[tris(3-t-butyl-phenylphosphine)]dichloride, cobalt bis[tris(3,5-diethylphenylphosphine)] dichloride, cobalt bis[tris(3-methyl-5-ethylphenylphosphine)]dichloride, cobalt bis[tris(3-phenylphenylphosphine)]dichloride, cobalt bis[tris(3,4,5-trimethylphenylphosphine)]dichloride, cobalt bis[tris(4-methoxy-3,5-dimethylphenylphosphine)]dichloride, cobalt bis[tris(4-ethoxy-3,5-diethylphenylphosphine)]dichloride, cobalt bis[tris(4-butoxy-3,5-dibutylphenylphosphine)]dichloride, cobalt bis[tris(4-methoxyphenylphosphine)]dichloride, cobalt bis[tris(3-methoxyphenylphosphine)]dichloride, cobalt bis[tris(4-dodecylphenylphosphine)]dichloride and cobalt bis[tris(4-ethylphenylphosphine)] dichloride.

Of these, particularly preferred are cobalt bis(triphenylphosphine)dichloride, cobalt bis[tris(3-methylphenylphosphine)]dichloride, cobalt bis[tris(3,5-dimethylphenyl-phosphine)]dichloride and cobalt bis[tris(4-methoxy-3,5-dimethylphenylphosphine)]dichloride.

As the amount of the catalyst used, the cobalt compound is used in an amount of 0.001 to 1 mmol, preferably about 0.01 to about 0.5 mmol, in terms of a cobalt atom per mole of butadiene for homopolymerization of butadiene, and per mole of the total amount of butadiene and a conjugated diene other than butadiene for copolymerization. Further, the amount of the phosphine compound used is usually from 0.1 to 50, preferably from 0.5 to 20, and more preferably from 1 to 20, as the atomic ratio of phosphorus to cobalt (P/Co) . Furthermore, the amount of the aluminoxane used is usually from 4 to $10^7$, and preferably from 10 to $10^6$, as the atomic ratio of aluminum to cobalt of the cobalt compound (Al/Co). When the complex represented by general formula (IV) is used, the amount of the phosphine compound used is 2 as the atomic ratio of phosphorus to cobalt (P/Co), and the amount of the aluminoxane used follows the above description.

Inert organic solvents used as polymerization solvents include, for example, aromatic hydrocarbon solvents such as benzene, toluene, xylene and cumene, aliphatic hydrocarbon solvents such as n-pentane, n-hexane and n-butane, alicyclic hydrocarbon solvents such as cyclopentane, methylcyclopentane and cyclohexane, and mixtures thereof.

The polymerization temperature is usually from -50 to 120˚C, and preferably from -20 to 100˚C. The polymerization reaction may be either batch-wise or continuous. The monomer concentration in the solvent is usually from 5 to 50% by weight, and preferably from 10 to 35% by weight.

Further, for producing the polymer, it is necessary to take into consideration that contamination with a compound having an inactivating function such as oxygen, water or carbon dioxide in a polymerization system should be decreased to the utmost, in order not to inactivate the catalyst and polymer of the invention. When the polymerization reaction proceeds to a desired stage, an alcohol, another polymerization terminator, an antiaging agent, an antioxidant, an ultraviolet absorber and the like are added to the reaction mixture, and then, the polymer formed is separated, washed and dried according to conventional methods. Thus, the syndiotactic 1,2-polybutadiene used in the present invention can be obtained.

The weight average molecular weight of (A) the syndiotactic 1,2-polybutadiene used in the present invention is preferably from 10,000 to 5,000,000, more preferably from 10,000 to 1,500,000, and particularly preferably from 50,000 to 1, 000, 000. When the weight average molecular weight is less than 10,000, fluidity is extremely high, unfavorably resulting in very difficult processing and the formation of a sticky formed article (medical member). On the other hand, exceeding 5,000,000 results in extremely low fluidity, which unfavorably makes processing very difficult.

On the other hand, (B) the thermoplastic polymer is a thermoplastic resin and/or thermoplastic elastomer other than the above-mentioned component (A), and specifically, at least one selected from the group consisting of polyethylene, polypropylene, a styrene-butadiene-styrene block copolymer (SBS), a styrene-isoprene-styrene block copolymer (SIS), hydrogenated polymers thereof (SEBS and SEPS), polybutadiene (BR) other than the above-mentioned syndiotactic 1,2-polybutadiene, an ABS resin, polyisoprene, various polyethylenes (LLDPE, ULDPE and LDPE), an ethylene-vinyl acetate copolymer, an ethyleneacrylate copolymer and an ethylene-methacrylate copolymer.

The amount of component (B) blended is 40 parts by weight or less, and preferably from 0 to 35 parts by weight, based on 100 parts by weight of the total amount of components (A) and (B). Exceeding 40 parts by weight results in a decrease in the ratio of component (A) used. As a result, the original flexibility of component (A) is lost.

Further, the composition used in the present invention may contain an additive such as a lubricant, a filler or a foaming agent, in addition to the above-mentioned components (A) and (B), as needed.

Specific examples of the above-mentioned additives include fillers such as talc, silica, magnesium hydroxide, calcium carbonate, glass, carbon fiber and glass balloons, and foaming agents such as Microsphere manufactured by Matsumoto Yushi-Seiyaku Co., Ltd., ADCA, OBSH, sodium bicarbonate and AIBN, as well as lubricants such as paraffin oil, silicone oil, liquid polyisoprene, liquid butadiene, erucic acid amide and stearic acid amide.

The amount of the lubricant used is 10 parts by weight or less, and preferably from 0.01 to 8 parts by weight, based on 100 parts by weight of the resin components, namely the total of components (A) and (B). When it exceeds 10 parts by weight, the lubricant unfavorably bleeds out from a product to seep into a drug used.

Further, in order to improve the balance between heat resistance and flexibility by electron beam irradiation, another additive, for example, a multifunctional monomer such as trimethylolpropane trimethacrylate, a photopolymerization initiator such as hydroxycyclohexyl phenyl ketone, or a photosensitizer such as benzophenone may be added in an amount of 5 parts by weight or less based on 100 parts by weight of the syndiotactic 1,2-polybutadiene.

Preparation and Forming of Composition

The composition used in the present invention, which is the above-mentioned component (A) alone or components (A) and (B) or a composition containing the above-mentioned additive (s) added thereto as needed, is softened

by heating, kneaded and formed. Kneading and forming are conducted at a temperature equal to or higher than the softening temperature or melting temperature of the syndiotactic 1,2-polybutadiene, at which good formability is exhibited, thereby providing a homogeneous formed article (medical member). Accordingly, the forming temperature is preferably from about 90 to about 170°C.

In order to obtain the formed articles such as the tube and the connecting member, there is utilized press molding, extrusion molding, injection molding, blow molding, profile extrusion molding, T-die film molding, inflation molding, powder slush molding, rotational molding or the like.

Electron Beam Irradiation

Of the medical members of the present invention, the tube requires flexibility, so that low crystalline RB is used. However, the melting point thereof is low. Accordingly, in order to cause it to exhibit steam sterilization resistance, it can be crosslinked by subsequent electron beam irradiation. The electron beam irradiation forms a three-dimensional crosslinked structure by radical polymerization of a vinyl group of the syndiotactic 1,2-polybutadiene to cure the formed article (tube) and give heat resistance. An electron beam has permeability to a synthetic resin, and the degree of its permeation depends on the thickness of a formed article and kinetic energy of the electron beam.

When energy of the electron beam is controlled permeably uniformly in the thickness direction according to irradiation thickness, the formed article (tube) whose degree of crosslinking is homogenized in the thickness direction can be obtained.

The connecting member may also be subjected to the electron beam irradiation.

The electron beam irradiation may be carried out either before or after adhesion of the tube to the connecting member.

The energy of the electron beam is preferably from 50 to 3,000 kV, and more preferably from 300 to 2,000 kV, to the above-mentioned medical member. The energy less than 50 kV results in a relative increase in the ratio of electrons captured and absorbed by a surface layer to decrease the electron beam which permeates through the medical member. Accordingly, the inside thereof is crosslinked late compared to the surface layer, which unfavorably causes the difference in the degree of crosslinking. On the other hand, the energy exceeding 3, 000 kV results in too high a degree of crosslinking, thereby making the medical member so hard as to unfavorably cause low elasticity and elongation.

Further, the dose of the electron beam in this case is preferably from 1 to 100 Mrad (corresponding to 10 to 1,000 kGy in the SI unit system), and more preferably from 1 to 50 Mrad. The medical member is irradiated with the electron beam within this dose range to perform curing by crosslinking. Less than 1 Mrad results in a decrease in the degree of crosslinking of 1,2-polybutadiene, whereas exceeding 100 Mrad results in too high a degree of crosslinking, thereby making the medical member hard, which unfavorably causes low elasticity and elongation.

The effect of crosslinking by electron beam irradiation can be expressed by the product of electron beam energy and dose. In the present invention, the product of electron beam acceleration voltage (kV) and irradiation dose (Mrad) is preferably from 2,000 to 20,000 (kV·Mrad), and more preferably from 5,000 to 16,000 (kV-Mrad). The product less than 2,000 (kV·Mrad) results in a relative increase in the ratio of electrons captured and absorbed by a surface layer to decrease the electron beam which permeates through the medical member. Accordingly, the inside thereof is crosslinked late compared to the surface layer, which unfavorably causes the difference in the degree of crosslinking. On the other hand, the product exceeding 20,000 (kV·Mrad) results in too high a degree of crosslinking, thereby making the medical member so hard as to unfavorably cause low elasticity and elongation.

The degree of elasticity at an elongation of 50% of the medical member after the electron beam irradiation ($M2_{50}$) can be increased to preferably 1.1 to 2.5 times, more preferably 1.1 to 2.0 times the degree of elasticity at an elongation of 50% before the electron beam irradiation ($M1_{50}$) by applying the electron beam irradiation described above to the medical member of the present invention. When $M2_{50}/M1_{50}$ is less than 1.1, the electron beam crosslinking does not proceed, resulting in poor steam sterilization resistance. On the other hand, when it exceeds 2.5, the medical member crosslinked becomes too hard, unfavorably resulting in the loss of flexibility. $M2_{50}/M1_{50}$ can be easily controlled by adjusting the above-mentioned product of electron beam acceleration voltage (kV) and irradiation dose (Mrad) to 2,000 to 20,000 (kV·Mrad).

The thus-obtained medical member crosslinked after the electron beam irradiation has steam sterilization resistance. For example, even when the crosslinked infusion tube of the present invention is sterilized by steam at a temperature of 100 to 121°C for about 10 to about 20 minutes, it does not happen to deform.

Specifically, the term "steam sterilization resistance" as used herein means that when a formed article of a resin such as the infusion tube (for example, a tube having an internal diameter of 3 mm, an outer diameter of 4.4 mm, a wall thickness of 0. 7 mm and a tube length of 20 cm) is placed in a high-pressure steam sterilizer and sterilized by steam at 121°C for 20 minutes, the circular shape before sterilization is kept and no deformation is observed.

Further, the haze value of the medical member of the present invention irradiated with the electron beam is preferably 30 or less, and more preferably 25 or less. The haze value is the measure of transparency, and the transparency

is improved with a decrease in this value. This haze value was measured in accordance with ASTM D-1003.

Furthermore, the toluene insoluble of the medical member of the present invention after the electron beam irradiation is usually from 50 to 99% by weight, and preferably from 80 to 95% by weight. The toluene insoluble is the barometer showing to what degree double bonds in (A) the syndiotactic 1,2-polypropylene have been crosslinked.

The toluene insoluble was determined herein in the following manner:

The medical member of the present invention ((a) g) was immersed in 100 ml of toluene, and allowed to stand at 30˚C for 48 hours. Then, filtration was carried out using a 100-mesh filter. After a part ((c) ml) of the filtrate was collected, it was evaporated to dryness. The resulting residual solid matter (toluene insoluble: (b) g) was weighed, and the gel content was calculated by the following equation:

$$\text{Gel content (\% by weight)} = [\{a-b\times(100/c)\}/a]\times 100$$

When the toluene insoluble is less than 50% by weight, crosslinking by the electron beam irradiation is insufficient, which causes poor heat resistance to result in poor steam sterilization resistance. On the other hand, when it exceeds 99% by weight, crosslinking by the electron beam irradiation proceeds too much. As a result, the medical member becomes too hard, unfavorably resulting in the loss of flexibility.

The above-mentioned toluene insoluble can be easily controlled by adjusting the above-mentioned product of electron beam acceleration voltage (kV) and irradiation dose (Mrad) to 2,000 to 20,000 (kV·Mrad).

Further, the halogen atom content of the medical member of the present invention is preferably 200 ppm or less, and more preferably 100 ppm or less.

The halogen atom content of the resulting 1,2-polybutadiene can be adjusted preferably to 200 ppm or less, and more preferably to 100 ppm or less, for example, by using the inert organic solvent of the non-halogen family as the polymerization solvent, as described above. Further, it is preferred that only non-halogen compounds are used in the catalyst system, because the halogen atom content of the medical member can be further reduced.

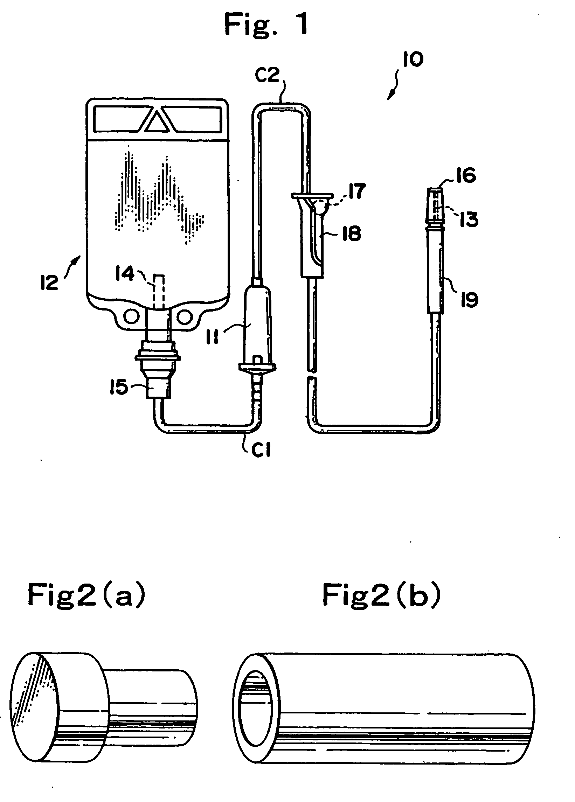

An infusion set using the medical members of the invention will be illustrated in greater detail below with reference to Fig. 1.

The infusion set 10 comprises a closure-piercing device 14 with a tube connecting portion 15 for discharging an infusion solution in an infusion bag 12, a drip chamber 11, a first tube C1 for connecting the closure-piercing device 14 to the drip chamber 11, a puncture needle 13, a second tube C2 for connecting the drip chamber 11 to the puncture needle 13, a connector 19 for connecting the second tube C2 to the puncture needle 13, a roller clamp 18 with a roller 17 intervening along the second tube C2 for adjusting the infusion rate, and a cap 16 for covering the puncture needle 13.

As the puncture needle 13, there is used a hollow metal needle made of stainless steel or a hollow needle made of a synthetic resin, which has a cutting edge for puncture at its tip. Further, as the roller clamp 18, there is used a roller clamp to which the roller 17 is movably attached. The movement of the roller 17 to the side of the puncture needle 13 narrows a flow path of the second tube C2, thereby being able to adjust the infusion rate. For an emergency that foreign matter is contained in a transfusion, a filter (not shown) is contained in the drip chamber 11. As the puncture needle 13, there is used one which has hitherto been used.

Further, in the present invention, all of the closure-piercing device 14 with the tube connecting portion 15, the drip chamber 11 and the connector 19 correspond to the connecting member specified in the present invention, and the syndiotactic 1, 2-polybutadiene having a crystallinity of 25 to 40% is used.

Further, as the tubes C1 and C2, soft tubes having transparency are suitable. Specifically, one obtained by electron beam irradiation of the syndiotactic 1,2-polybutadiene of the present invention having a crystallinity of 5 to 25% is used instead of a soft vinyl chloride resin or syndiotactic 1,2-polybutadiene having a low crystallinity of about 5 to about 25% which has hitherto been used.

Respective ends of the tubes C1 and C2 are firmly adhered and fixed to the connecting members such as the closure-piercing device 14 with the tube connecting portion 15, the drip chamber 11 and the connector 19 by solvent adhesion, ultrasonic adhesion or high-frequency adhesion.

In the present invention, the tubes and the connecting members are all formed of the syndiotactic 1,2-polybutadiene, so that the tubes can be firmly adhered and fixed to the connecting members, which causes no leak. Solvents used herein for solvent adhesion include tetrahydrofuran, cyclohexane and toluene.

Adhesion processes include adhesive adhesion, ultrasonic adhesion and high-frequency adhesion, as well as solvent adhesion.

In the present invention, the medical member comprising the tube(s) and the connecting member(s) can also be applied as a constituent element for the above-mentioned infusion set or a constituent element for the medical instrument such as a catheter for pharmaceutical administration.

According to the present invention, there can be provided the medical member mainly comprising the syndiotactic 1, 2-polybutadiene, which is useful for medical use, is excellent in flexibility and hardness, is also excellent in steam sterilization resistance, has no leak at a joint, is recyclable, and is further environmentally friendly because it contains no vinyl chloride resin, and the medical instrument using the same.

The present invention will be illustrated in greater detail with reference to the following examples, but the invention should not be construed as being limited thereto. In the examples, parts and percentages are by weight, unless otherwise specified. Further, various measurements in the examples were made according to the following:

Reference Example 1

The following were used as tubes.

Tube A-1: RB having a crystallinity of 18% (JSR RB810 manufactured by JSR Corporation)
Tube A-2: A blend of RB having a crystallinity of 24% (JSR RB820 manufactured by JSR Corporation) and SIS (a styrene-isoprene-styrene block copolymer, JSR SIS5229P manufactured by JSR Corporation) (weight ratio = 70/30)
Tube A-3: An electron beam (EB) irradiated product of RB for tube A-1 (500 kV X 5 Mrad)
Tube A-4: An electron beam (EB) irradiated product of the blend for tube A-2 (500 kV X 5 Mrad)
Tube a-1: A blend of 100 parts of LLDPE (LUMITAC 24-1 manufactured by Tosoh Corporation) and 15 parts of a plasticizer (liquid paraffin)
Tube a-2: Plasticized PVC (Daiichi PVC SX-13 manufactured by Sumitomo Chemical Co., Ltd. (containing 60 phr of DOP))

All of the tubes have an internal diameter of 3 mm, an outer diameter of 4.4 mm, a wall thickness of 0.7 mm and a tube length of 20 cm).

Properties of the respective tubes are shown in Table 1.

Reference Example 2

The following were used as connectors.

Connector B-1: RB840 manufactured by JSR Corporation (syndiotactic 1, 2-polybutadiene having a crystallinity of 34%)
Connector B-2: An EB irradiated product of RB840 described above (500 kV X 5 Mrad)
Connector B-3: RB830 manufactured by JSR Corporation (syndiotactic 1, 2-polybutadiene having a crystallinity of 28%)
Connector b-1: A polypropylene resin for injection molding (Polypro J5080Q manufactured by Tosoh Corporation)
Connector b-2: A polycarbonate resin for injection molding (Panlite K-1285J manufactured by Teijin Chemicals Ltd.)
Connector b-3: PVC for injection molding (Daiich PVC SX-8G manufactured by Sumitomo Chemical Co., Ltd.)

Properties of the respective connectors are shown in Table 1.

All of the connectors have an outer diameter of an adhering portion of 3.7 mm, and a length of an adhering portion of 1 cm.

Each connector described above and each tube described in Reference Example 1 are schematically shown in Fig. 2, in which (a) indicates the connector and (b) indicates the tube.

Table 1

| Various Properties of Tubes and Connectors | | | |
|---|---|---|---|
| | | Halogen Content | Plasticizer |
| Tube | | | |
| A-1 | RB | O | O |
| A-2 | RB/SIS | O | O |
| A-3 | EB Irradiated Product of A-1 | O | ○ |
| A-4 | EB Irradiated Product of A-2 | O | ○ |
| a-1 | LLDPE/Plasticizer | O | × |

(continued)

| Various Properties of Tubes and Connectors | | | |
|---|---|---|---|
| | | Halogen Content | Plasticizer |
| Tube | | | |
| a-2 | Plasticized PVC | × | × |
| Connector | | | |
| B-1 | RB 840 | O | O |
| B-2 | EB Irradiated Product of RB840 | O | O |
| B-3 | RB830 | O | O |
| b-1 | PP | ○ | ○ |
| b-2 | PC | ○ | ○ |
| b-3 | PVC | × | ○ |

Halogen Content: A tube or a connector having a halogen content of 200 ppm or less was evaluated as O, and a tube or a connector having a halogen content exceeding 200 ppm was evaluated as ×.
Plasticizer: A tube or a connector containing no plasticizer was indicated as O, and a tube or a connector containing a plasticizer was indicated as ×.

Examples 1 to 12 and Comparative Examples 1 to 24

The tubes of Reference Examples 1 were combined with the connectors of Reference Examples 2 to prepare medical members, which were each evaluated for (1) solvent adhesiveness, (2) steam sterilization resistance and (3) ethylene oxide gas (EOG) sterilization resistance. The results thereof are shown in Table 2.

Table 2 (Evaluations of Tube/Connector Combinations)

| Evaluation Item | | |
|---|---|---|
| 1 | 2 | 3 |
| 1: Solvent Adhesiveness (○: good, × : poor)<br>2: Steam Sterilization Resistance (○: good, × : poor)<br>3: EOG Sterilization Resistance (○: good, ×: poor) | | |

Results of Joining Tests of Tube and Connector

| | | B-1 | | | B-2 | | | B-3 | | | b-1 | | | b-2 | | | b-3 | |
|---|---|---|---|---|---|---|---|---|---|---|---|---|---|---|---|---|---|---|
| A-1 | Example 1 | | | Example 2 | | | Example 3 | | | Comparative Example 1 | | | Comparative Example 2 | | | Comparative Example 3 | | |
| | ○ | × | ○ | ○ | × | ○ | ○ | × | ○ | × | × | ○ | × | × | ○ | × | × | × |
| A-2 | Example 4 | | | Example 5 | | | Example 6 | | | Comparative Example 4 | | | Comparative Example 5 | | | Comparative Example 6 | | |
| | ○ | × | ○ | ○ | × | ○ | ○ | × | ○ | × | × | ○ | × | × | ○ | × | × | ○ |
| A-3 | Example 7 | | | Example 8 | | | Example 9 | | | Comparative Example 7 | | | Comparative Example 8 | | | Comparative Example 9 | | |
| | ○ | ○ | ○ | ○ | ○ | ○ | ○ | × | ○ | × | ○ | ○ | × | ○ | ○ | × | ○ | ○ |
| A-4 | Example 10 | | | Example 11 | | | Example 12 | | | Comparative Example 10 | | | Comparative Example 11 | | | Comparative Example 12 | | |
| | ○ | ○ | ○ | ○ | ○ | ○ | ○ | × | ○ | × | ○ | ○ | × | ○ | ○ | × | ○ | ○ |
| a-1 | Comparative Example 13 | | | Comparative Example 14 | | | Comparative Example 15 | | | Comparative Example 16 | | | Comparative Example 17 | | | Comparative Example 18 | | |
| | × | ○ | ○ | × | ○ | ○ | × | × | ○ | × | ○ | ○ | × | ○ | ○ | × | ○ | ○ |
| a-2 | Comparative Example 19 | | | Comparative Example 20 | | | Comparative Example 21 | | | Comparative Example 22 | | | Comparative Example 23 | | | Comparative Example 24 | | |
| | × | ○ | ○ | × | ○ | ○ | × | × | ○ | × | ○ | ○ | × | ○ | ○ | ○ | ○ | ○ |

1. Solvent Adhesiveness: A tube was adhered to a connector by solvent adhesion using cyclohexane as a solvent. An end portion of the tube on the opposite side of the adhering portion thereof was bent and fixed with a clip. Then, compressed air (0.3 MPa) was supplied from the connector side to the tube, and it was observed in water whether an air leak occurred or not. The solvent adhesiveness was evaluated according to the following criteria:

Good (○): No air leak was observed.
Poor (×) : An air leak was observed.

2. Steam Sterilization Resistance: The steam sterilization resistance was evaluated according to the following criteria:

(○): No change in shape was observed after steam sterilization (121˚C X 20 minutes).
( × ): A change in shape was observed after steam sterilization (121˚C X 20 minutes).

3. EOG Sterilization Resistance: The EOG sterilization resistance was evaluated according to the following criteria:

(○): No change in shape was observed after steam sterilization (60˚C X 120 minutes).
( × ): A change in shape was observed after steam sterilization (60˚C X 120 minutes).

Disclosed are a medical member having a tube mainly containing syndiotactic 1,2-polybutadiene having a crystallinity of 5 to 25% and a connecting member such as a connector connected thereto, the connecting member mainly containing syndiotactic 1,2-polybutadiene having a crystallinity of 25 to 40%, and a medical instrument such as an infusion set having the medical member as a constituent element. The medical member is excellent in flexibility and hardness, has no leak at a joint, and is also excellent in steam sterilization resistance.

## Claims

1. A medical member comprising a tube mainly comprising syndiotactic 1,2-polybutadiene having a crystallinity of 5% to 25% and a connecting member connected thereto, the connecting member mainly comprising syndiotactic 1,2-polybutadiene having a crystallinity of 25 to 40%.

2. The medical member according to claim 1, wherein the connecting member is at least one selected from the group consisting of a closure-piercing device, a drip chamber, a graduated buret, an air trap, an injection site, a three-way cock and a connector.

3. The medical member according to any one of claims 1 and 2, wherein the tube is connected to the connecting member by solvent adhesion, ultrasonic adhesion or high-frequency adhesion.

4. The medical member according to any one of claims 1 to 3, which is sterilizable by steam.

5. The medical member according to any one of claims 1 to 4, wherein the tube is crosslinked by electron beam irradiation.

6. The medical member according to claim 5, wherein the product of electron beam acceleration voltage (kV) and irradiation dose (Mrad) used in the crosslinking is from 2,000 to 20,000 (kV.Mrad).

7. The medical member according to any one of claims 1 to 6, wherein the halogen content is 200 ppm or less.

8. The medical member according to any one of claims 1 to 7, wherein a lubricant is contained in an amount of 10 parts by weight or less based on 100 parts by weight of a resin component mainly comprising syndiotactic 1,2-polybutadiene.

9. A medical instrument having the medical member according to any one of claims 1 to 8 as a constituent element.

## Patentansprüche

1. Medizinisches Element, umfassend eine Röhre, die hauptsächlich syndiotaktisches 1,2-Polybutadien mit einer Kristallinität von 5% bis 25% umfasst, und ein verbindendes Element, das damit verbunden ist, wobei das verbindende Element hauptsächlich syndiotaktisches 1,2-Polybutadien mit einer Kristallinität von 25 bis 40% umfasst.

**2.** Medizinisches Element nach Anspruch 1, wobei das verbindende Element mindestens eines, gewählt aus der Gruppe bestehend aus einer einen Verschluss durchbohrenden Vorrichtung, einer Tropfkammer, einer mit einer Gradeinteilung versehenen Bürette, einer Geruchschleuse, einer Injektionsstelle, einem Dreiwegehahn und einem Verbinder ist.

**3.** Medizinisches Element nach einem der Ansprüche 1 und 2, wobei die Röhre mit dem verbindenden Element durch Lösungsmitteladhäsion, Ultraschalladhäsion oder Hochfrequenzadhäsion verbunden ist.

**4.** Medizinisches Element nach einem der Ansprüche 1 bis 3, das durch Dampf sterilisierbar ist.

**5.** Medizinisches Element nach einem der Ansprüche 1 bis 4, wobei die Röhre durch Elektronenstrahl-Bestrahlung vernetzt ist.

**6.** Medizinisches Element nach Anspruch 5, wobei das Produkt aus der im Vernetzen verwendeten Elektronenstrahlbeschleunigungsspannung (kV) und der Bestrahlungsdosis (Mrad) von 2000 bis 20000 (kV.Mrad) ist.

**7.** Medizinisches Element nach einem der Ansprüche 1 bis 6, wobei der Halogenanteil 200 ppm oder weniger ist.

**8.** Medizinisches Element nach einem der Ansprüche 1 bis 7, wobei ein Schmiermittel enthalten ist in einer Menge von 10 Gewichtsteilen oder weniger, basierend auf 100 Gewichtsteilen eines Harzbestandteils, der hauptsächlich syndiotaktisches 1,2-Polybutadien umfasst.

**9.** Medizinisches Instrument mit dem medizinischen Element nach einem der Ansprüche 1 bis 8 als ein einen Teil bildendes Element.

## Revendications

**1.** Article médical comprenant un tube comprenant principalement du 1,2-polybutadiène syndiotactique ayant une cristallinité de 5% à 25% et un élément de raccord relié à celui-ci, l'élément de raccord comprenant principalement du 1,2-polybutadiène syndiotactique ayant une cristallinité de 25 à 40%.

**2.** Article médical selon la revendication 1, dans lequel l'élément de raccord est au moins un élément choisi dans le groupe constitué d'un dispositif de perforation de capuchon, une chambre compte-gouttes, une burette graduée, une prise d'air, un site d'injection, un robinet à trois voies et un raccord.

**3.** Article médical selon l'une quelconque des revendications 1 et 2, dans lequel le tube relié à l'élément de raccord par adhésion par un solvant, adhésion par ultrasons ou adhésion par haute-fréquence.

**4.** Article médical selon l'une quelconque des revendications 1 à 3, qui est stérilisable par la vapeur.

**5.** Article médical selon l'une quelconque des revendications 1 à 4, dans lequel le tube est réticulé par irradiation par un faisceau d'électrons.

**6.** Article médical selon la revendication 5, dans lequel le produit de la tension (kV) d'accélération du faisceau d'électrons et de la dose de l'irradiation (Mrad) utilisées dans la réticulation est de 2 000 à 20 000 (kV.Mrad).

**7.** Article médical selon l'une quelconque des revendications 1 à 6, dans lequel la teneur en halogène est de 200 ppm ou moins.

**8.** Article médical selon l'une quelconque des revendications 1 à 7, dans lequel un lubrifiant est contenu dans une quantité de 10 parties en poids ou moins, sur la base de 100 parties en poids d'un composant de résine comprenant principalement du 1,2-polybutadiène syndiotactique.

**9.** Instrument médical ayant l'article médical selon l'une quelconque des revendications 1 à 8 comme élément constitutif.

Fig. 1

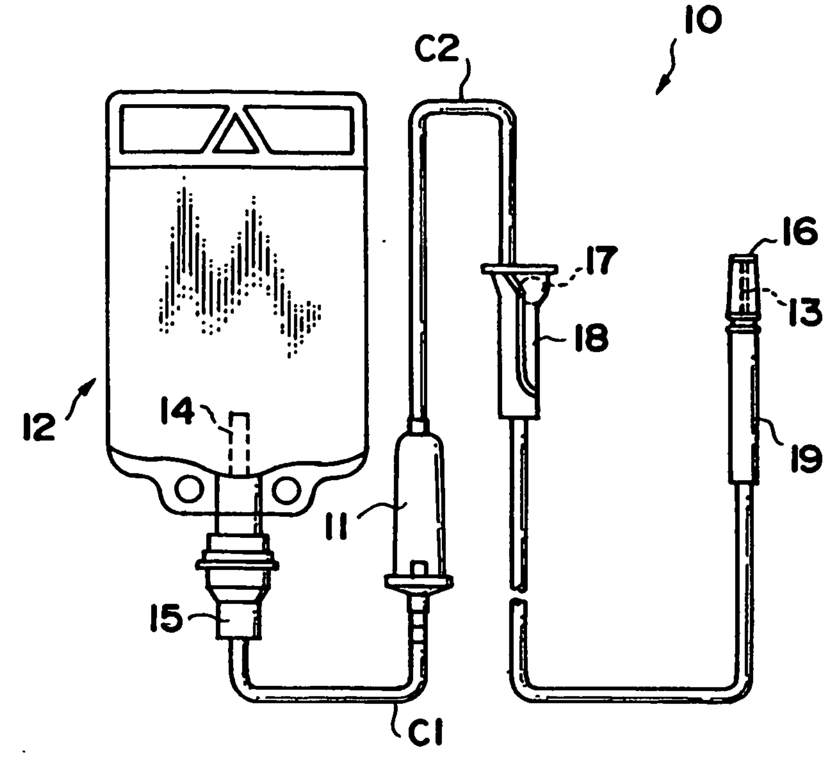


Fig2 (a)

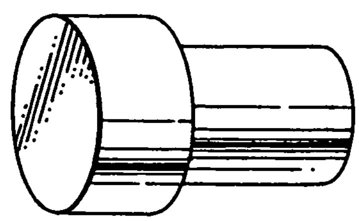

Fig2 (b)